# EUROPEAN PATENT SPECIFICATION

(11) **EP 0 822 808 B1**
(45) Date of publication and mention of the grant of the patent: **26.07.2000**
(21) Application number: 96913671.2
(22) Date of filing: 17.04.1996
(51) Int. Cl.: A61K 9/18, A61K 47/36

(54) **COMPOSITE WITH SODIUM STARCH GLYCOLATE AS A SUPPORT MATERIAL AND PRODUCTS THEREOF**
ZUSAMMENSETZUNG MIT NATRIUMSTÄRKEGLYCOLAT ALS GRUNDLAGENMATERIAL UND DARAUS HERGESTELLTE PRODUKTE
COMPOSE CONTENANT DU GLYCOLATE D'AMIDON DE SODIUM EN TANT QUE MATERIAU SUPPORT ET PRODUITS A BASE DE CE COMPOSE

(30) Priority: 20.04.1995 US 425434
(43) Date of publication of application: 11.02.1998
(73) Proprietor: EURAND INTERNATIONAL S.p.A., 20060 Pessano con Bornago (Milano) (IT)
(72) Inventor: CARLI, Fabio, Via del Follatoio, 12 I-34148 Trieste (IT); SHAH, Jatin, J. EON Labs Manufacturing, Inc., Laurelton, NY 11413 (US); SHINAL, Edward. C. EON Labs Manufacturing, Inc., Laurelton, NY 11413 (US)
(74) Representative: Gervasi, Gemma, Dr.
(86) International application number: IB9600492
(87) International publication number: WO9632931

(56) References cited:
- EP-A- 0 371 431
- GB-A- 2 153 678
- US-A- 3 966 899
- US-A- 5 130 140

## Description

### BACKGROUND OF THE INVENTION

The present invention relates to mechanically activated composites which are formed by high energy co-grinding a pharmaceutically active substance with a support material. In particular, the present invention relates to the formation of mechanically activated composites using sodium starch glycolate as support material. Such composites may be used to formulate drugs having substantially increased rates of dissolution and solubilization kinetics of the active substance when compared to the active substance per se or to drugs that are formulated by previously known techniques.

In the previously known techniques, poorly soluble active substances are milled to improve their dissolution rates. Typically, the active substances are milled independently, or occasionally with suitable support materials, in mills, such as hammer, pin, or airjet mills, etc. The support materials generally facilitate the desired physico-chemical transformations of the drug.

U.S. Patents 3,966,899 and 4,036,990 teach grinding mixtures of drugs and β-1,4-glucan to increase the dissolution rate of poorly soluble drugs.

In Japanese Patent 7986607, β-cyclodextrin is the support material used in the co-grinding, either alone or with other excipients, such as lactose, calcium phosphate and starch.

β-cyclodextrin is also used in DE Patent 3427788 with benzimidazole derivatives by grinding with a ball mill.

The use of microcrystalline cellulose is described in Chem. Pharm. Bull., 78, 3340-6, 1977; Chem. Pharm. Bull., 78, 3419-25, 1978 and Chem. Pharm. Bull., 28, 652-6, 1980.

In EP Patent 129893, silica gel and other adsorbent materials are used with drugs such as griseofulvin, chloramphenicol and theophylline.

U.S. Patent 4,639,370 describes the use of polymers that are swellable but insoluble in water, such as cross-linked polyvinylpyrrolidone, cross-linked sodium carboxymethylcellulose and dextran with drugs such as medroxyprogesterone acetate, griseofulvin and indomethacin.

There is, however, need for a cost-effective support material to be used with a poorly soluble pharmaceutically active substance in a high energy co-grinding process to form a mechanically activated composite which provides greatly enhanced characteristics in drugs that are formulated with the composite.

### SUMMARY OF THE INVENTION

An object of the invention is to provide mechanically activated composites of sodium starch glycolate and pharmaceutically active substances, with enhanced dissolution rates and solubilization kinetics of the active substances when compared to the active substances per se or to formulations that are prepared using previously known techniques. The composites are obtained by co-grinding poorly soluble pharmaceutically active substances with sodium starch glycolate as a support material in a high energy mill.

Another object of the invention is to provide a process and apparatus for preparing mechanically activated composites by high energy co-grinding poorly soluble pharmaceutically active substances with sodium starch glycolate.

Yet another object of the invention is to provide a method of masking the taste of a pharmaceutically active substance by high energy co-grinding the active substance with sodium starch glycolate.

These and other objects of the invention are achieved by a unique mechanically activated composite comprising a pharmaceutically active substance and sodium starch glycolate as a support material for the active substance. The composite is prepared by high energy co-grinding a mixture of the active substance and the sodium starch glycolate to obtain the mechanically activated composite having a substantially increased dissolution rate, enhanced solubilization kinetics, greater bioavailability, and an improved peak drug level in plasma of the pharmaceutically active substance when compared to the active substance per se or to previously known formulations thereof.

Other aspects of the invention provide a process for manufacturing a mechanically activated composite and an apparatus therefor.

Yet another aspect of the invention provides a method of masking the taste of a pharmaceutically active substance by forming a mechanically activated composite of the active substance and sodium starch glycolate.

Other features and advantages of the present invention will become apparent from the following description of the invention which refers to the accompanying drawings.

### BRIEF DESCRIPTION OF THE DRAWINGS

Figure 1 is a graph showing comparative dissolution rates for conventionally ground ibuprofen-sodium starch glycolate (A and B) and high energy co-ground ibuprofen-sodium starch glycolate composite (C);
Figures 2A and 2B show SEM micrographs of sodium starch glycolate at 2500X and 5000X, respectively;
Figures 3A and 3B show SEM micrographs of ibuprofen at 2500X and 5000X, respectively;
Figures 4A and 4B show SEM micrographs of 1:1 ibuprofen-sodium starch glycolate mechanically activated composite after 120 minutes of high energy grinding at 2500X and 5000X, respectively;
Figures 5A to 5D show SEM micrographs of 1:1 ibuprofen-sodium starch glycolate mechanically activated composite after 30 minutes of high energy grinding at 250X, 1000X, 2500X and 5000X, respectively;
Figure 6 is a mean data graph of plasma ibuprofen concentrations for tablet formulations including 1:1 ibuprofen-sodium starch glycolate mechanically activated composite (A); 1:0.5 ibuprofen-sodium starch glycolate mechanically activated composite (B); and a prior art ibuprofen tablet formulation (W);
Figure 7 is a graph showing comparative dissolution rates for a prior art ibuprofen formulation (A and B) and a formulation including 1:1 ibuprofen-sodium starch glycolate mechanically activated composite (C and D); and
Figure 8 is a table showing comparative dissolution data for a prior art formulation (A) and a formulation (E) including 1:1 ibuprofen-sodium starch glycolate mechanically activated composite.

### DETAILED DESCRIPTION OF PREFERRED EMBODIMENTS OF THE INVENTION

The invention provides unique mechanically activated composites, which are formed by high energy co-grinding poorly soluble pharmaceutically active substances and sodium starch glycolate and have considerable unexpected advantages over drugs that are formulated using known techniques. In the composites, the poorly soluble active substances are resting or supported on sodium starch glycolate. Poorly soluble active substances are pharmaceutically active substances that dissolve in gastrointestinal fluid to an extent that is less than adequate for clinical effect. For example, poorly soluble active substances require from about 30 parts or more of solvent for 1 part of solute.

The mechanically activated composites provide greatly enhanced characteristics to the drugs, such as substantially increased rates of dissolution, enhanced solubilization kinetics, greater bioavailability, and higher peak drug levels in plasma. Rate of dissolution and solubilization kinetics are known test methods by which performance of a drug or formulation can be evaluated.

The inventors have unexpectedly found that sodium starch glycolate is an advantageous support material because, as compared with previously known support materials, sodium starch glycolate is significantly less expensive, requires substantially less grinding time to obtain the desired results, and with high energy co-grinding forms a unique mechanically activated composite with otherwise poorly soluble pharmaceutically active substances. Sodium starch glycolate has a high swelling volume (granules swell up to 300% of their volume), and good wettability.

Surprisingly, sodium starch glycolate increases substantially the dissolution rates of the active substances without decreasing the enthalpy of the active substances that are present in the composite, or converting the active substances to amorphous form, i.e., decreasing their crystallinity. It has unexpectedly been found that sodium starch glycolate as a support material in a mechanically activated composite, is a superior alternative to cross-linked polyvinylpyrrolidone.

Without being bound by theory, the inventors have unexpectedly found that a unique mechanically activated composite is formed by high energy co-grinding sodium starch glycolate and a poorly soluble pharmaceutically active substance. The sodium starch glycolate and one or more such active substances are simultaneously ground in a suitable high energy grinding apparatus. High energy grinding refers to a process in which a high energy impact is obtained between two pieces of grinding media with drug and support material particles in between the media. As a result of the high energy, and the unique and unexpected properties of sodium starch glycolate, a mechanically activated composite is obtained in a relatively short period of time. It has been found that it is not possible to obtain such a mechanically activated composite in a practically useful period of time with conventional milling processes and support materials in the pharmaceutical art.

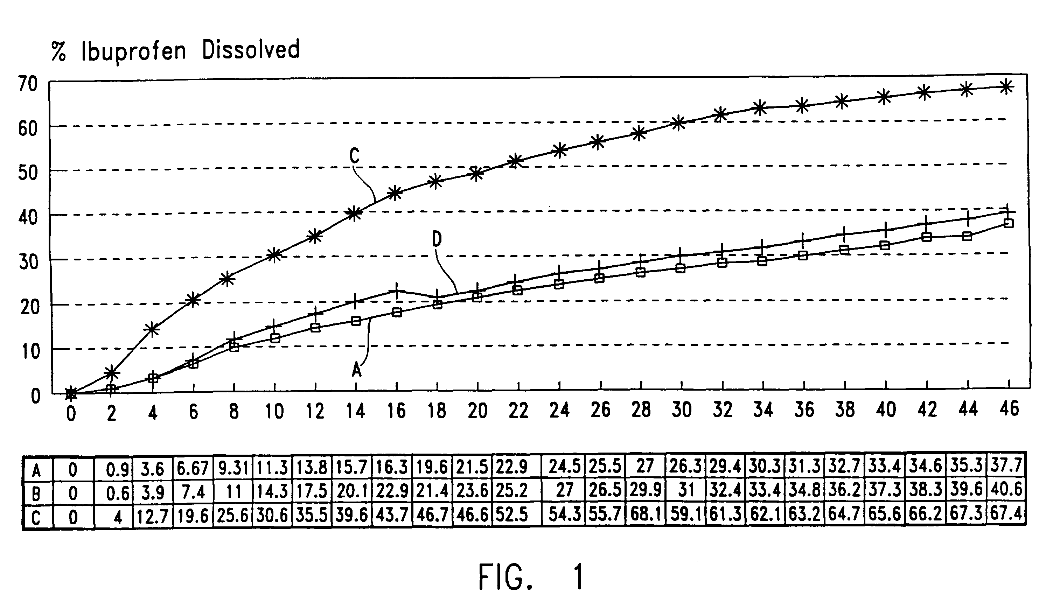

Figure 1 compares graphically the dissolution rates and gives the dissolution data for conventionally ground 1:1 ibuprofen-sodium starch glycolate (A and B) and a high energy co-ground 1:1 ibuprofen-sodium starch glycolate composite (C). A 1:1 mixture of ibuprofen and sodium starch glycolate was milled in a hammer mill (Fitzmill) equipped with a #0 screen (A) and a #000 screen (B), hammers forward, fast speed. A high energy vibration mill (Sweco DM24) was used to co-grind 1:1 ibuprofen-sodium starch glycolate for 30 minutes (C). The dissolution method utilized USP Apparatus II, 100rpm, 900 mL of pH 1.2 buffer.

The high energy co-ground ibuprofen-sodium starch glycolate composite C has significantly higher dissolution rates than the conventionally ground ibuprofen-sodium starch glycolate A and B.

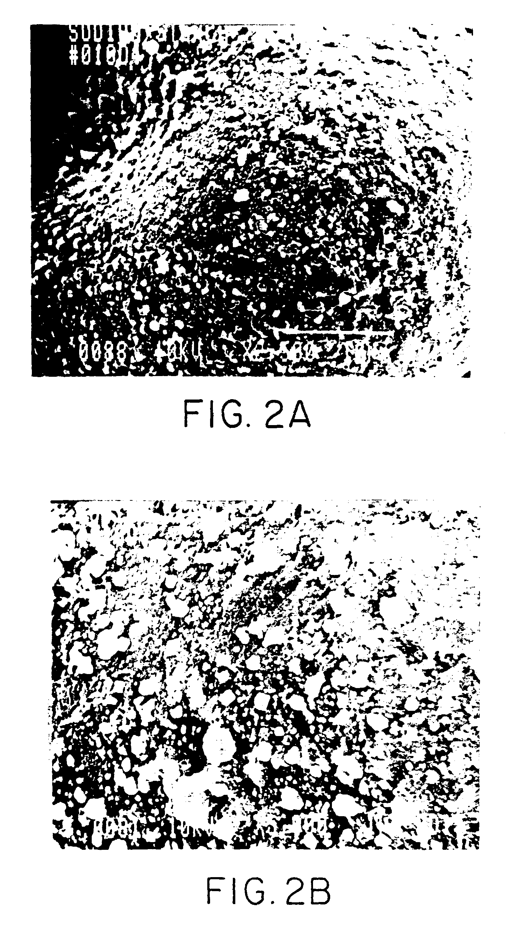

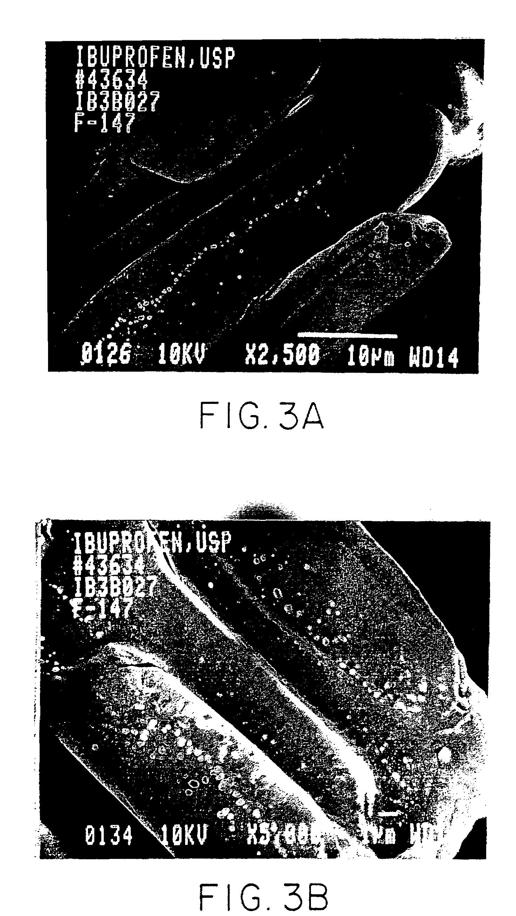

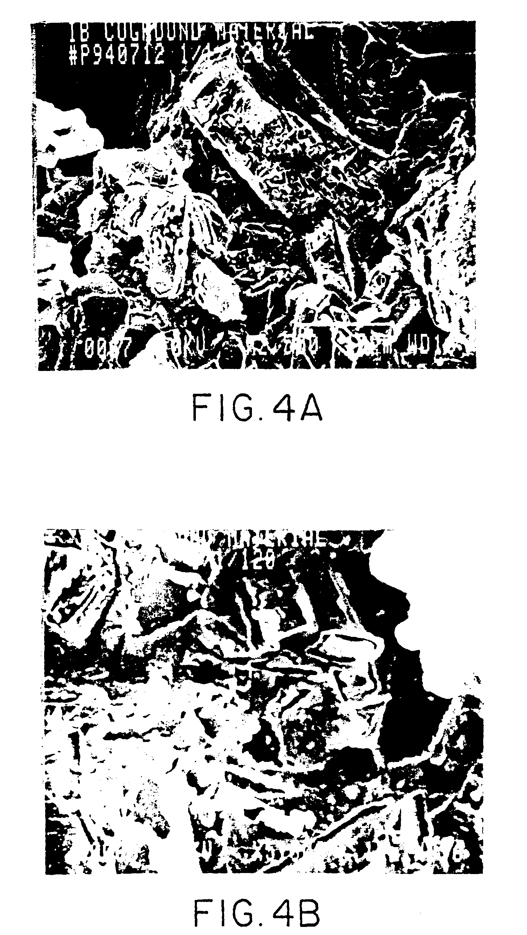

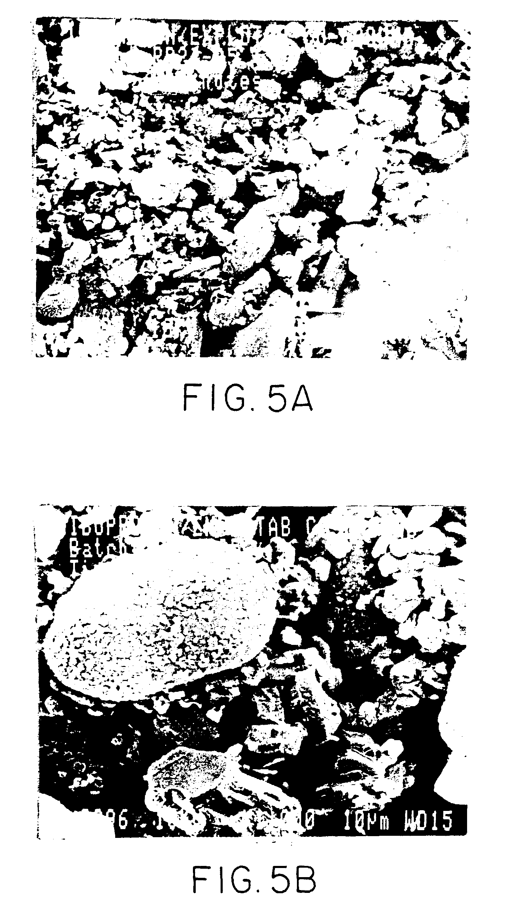

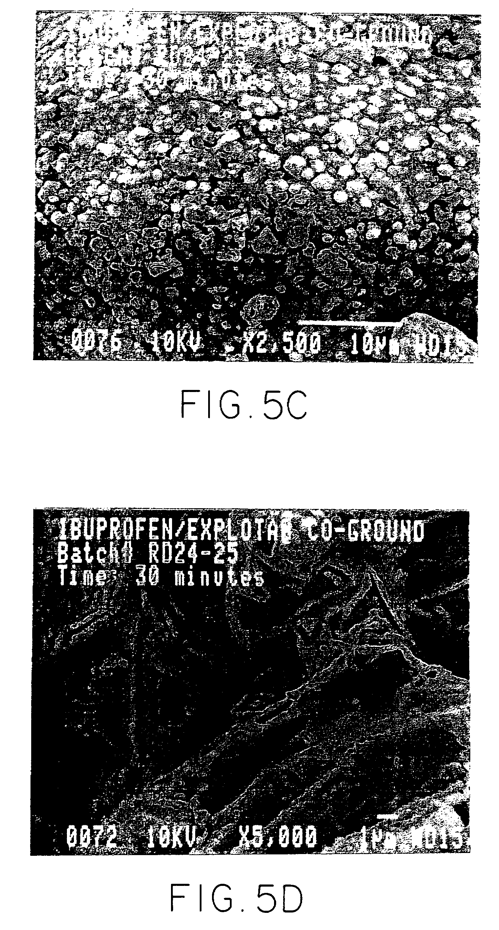

Figures 2A and 2B show SEM micrographs of sodium starch glycolate at 2500X and 5000X, respectively. Figures 3A and 3B show SEM micrographs of ibuprofen at 2500X and 5000X, respectively. Figures 4A and 4B show SEM micrographs of 1:1 ibuprofen-sodium starch glycolate mechanically activated composite after 120 minutes of high energy co-grinding at 2500X and 5000X, respectively, and Figures 5A to 5D show SEM micrographs of 1:1 ibuprofen-sodium starch glycolate mechanically activated composite after 30 minutes of high energy co-grinding at 250X, 1000X, 2500X and 5000X, respectively.

The above figures show that high energy co-grinding with sodium starch glycolate causes a change in the physical structures of the ibuprofen particles, however, without decreasing the crystallinity of the ibuprofen particles. It is believed that it may be the unique and unexpected properties of sodium starch glycolate, and the structure of the mechanically activated co-ground composite that results from high energy co-grinding which provide unexpected and advantageous results making sodium starch glycolate a preferred support material for poorly soluble drugs. It is also believed that swellability of sodium starch glycolate may lead to easier formation of the co-ground composite.

Because ibuprofen particles retain their crystallinity, it was expected the dissolution rate and solubilization kinetics characteristics in particular would remain unchanged. Surprisingly, however, substantially improved dissolution rates, solubilization kinetics, bioavailability, and peak drug levels in plasma of ibuprofen are obtained. Such improved results are generally not obtainable with other support materials known to the prior art without altering the enthalpy and peak melting point of the drug thereby possibly diffusing its physico-chemical properties and influencing biochemical characteristics.

Formulations that include a mechanically activated composite of a pharmaceutically active substance supported on sodium starch glycolate have substantially increased dissolution rates, solubilization kinetics, bioavailability, and peak drug levels in plasma of the active substance when compared to the active substance per se or to known formulations of the active substance that are formulated by existing techniques. With substantially reduced co-grinding times, drugs that include a mechanically activated composite having sodium starch glycolate as a support material have improved dissolution rates, solubilization kinetics, bioavailability, and peak drug levels in plasma compared with prior art drugs. Therefore, sodium starch glycolate offers advantageous efficiencies in the production of formulations of poorly soluble drugs.

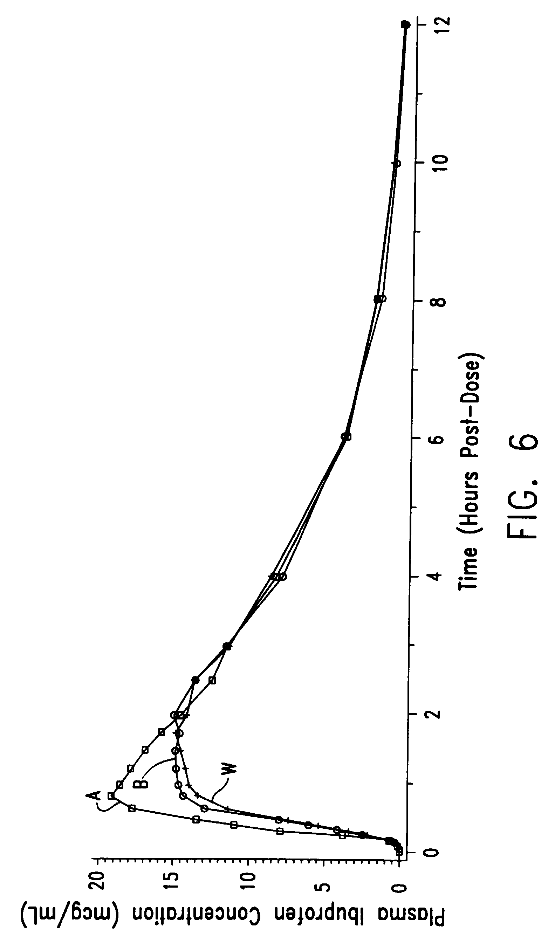

Figure 6 is a mean data graph of plasma ibuprofen concentrations (linear plot) for tablet formulations including 1:1 ibuprofen-sodium starch glycolate mechanically activated composite (A); 1:0.5 ibuprofen-sodium starch glycolate mechanically activated composite (B); and a prior art ibuprofen tablet formulation (W). The prior art ibuprofen tablet formulation W used for the comparison is Advil®. The tablet formulations A and B have the following compositions:

**Table I.**

| **Compositions of A and B 200 mg tablet formulations evaluated in Figure 6** | | | |
|---|---|---|---|
| Ingredient | | Quantity (mg per tablet) | |
| | | 1:1 | 1:0.5 |
| 1. | Ibuprofen USP | 200.0 | 200.0 |
| 2. | Sodium Starch Glycolate, NF | 200.0 | 100.0 |
| 3. | Microcrystalline Cellulose (PH 101) | 40.0 | 40.0 |
| 4. | Lactose, NF | 62.0 | 72.0 |
| 5. | Colloidal Silicone Dioxide, NF | 40.0 | 30.0 |
| 6. | Talc, NF | 8.0 | 8.0 |
| | Total Wt. | 550.0 | 450.0 |

Figure 6 shows that formulations A and B are absorbed into the body substantially faster and to a greater extent than formulation W. It is believed the plasma ibuprofen concentrations of formulation B are close to those of formulation W because the drug-to-carrier ratio in formulation B was less favorable.

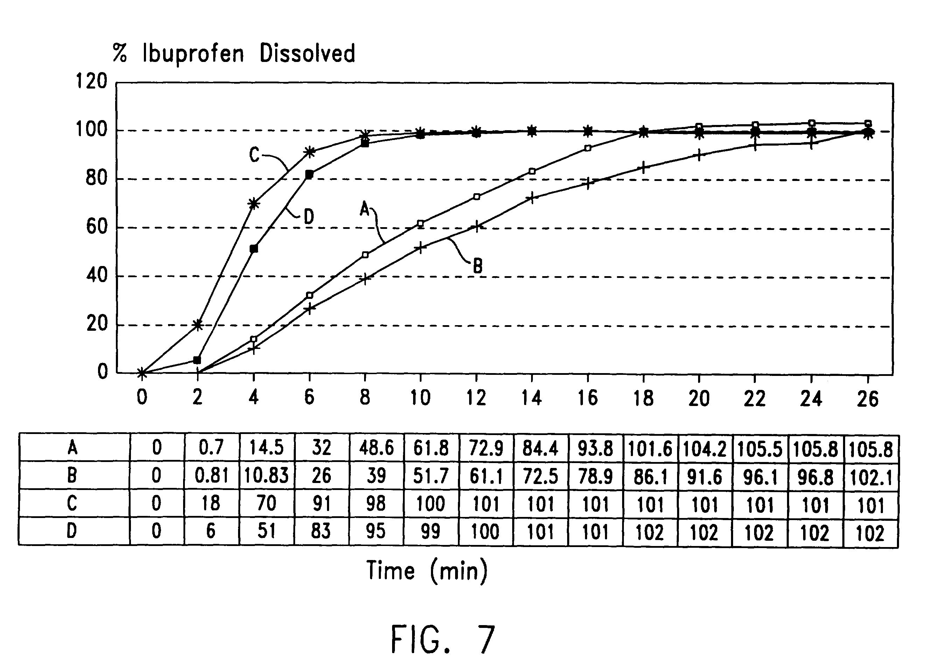

Figure 7 has a graph showing comparative dissolution rates and a table with dissolution data for a prior art ibuprofen formulation (A and B) and a formulation including 1:1 ibuprofen-sodium starch glycolate mechanically activated composite (C and D). The dissolution method utilized USP Apparatus II, 100 rpm, 900 mL of pH 5.5 buffer (A and C), and 50 rpm, 900 mL of pH 6.0 buffer (B and D). The prior art ibuprofen formulation used for A and B is Dolormin® and the formulation used for C and D has the following composition:

**Table II.**

| **Composition of C and D 200 mg tablet formulation evaluated in Figure 7** | | |
|---|---|---|
| Ingredient | | Quantity (mg per tablet) |
| 1. | Ibuprofen USP | 200.0 |
| 2. | Sodium Starch Glycolate, NF | 200.0 |
| 3. | Microcrystalline Cellulose | 30.0 |
| 4. | Crosscarmellose Sodium, NF | 50.0 |
| 5. | Colloidal Silicone Dioxide, NF | 5.0 |
| 6. | Stearic Acid, NF | 15.0 |
| | Total Wt. | 500.0 |

Figure 7 shows that C and D have substantially higher dissolution rates than A and B.

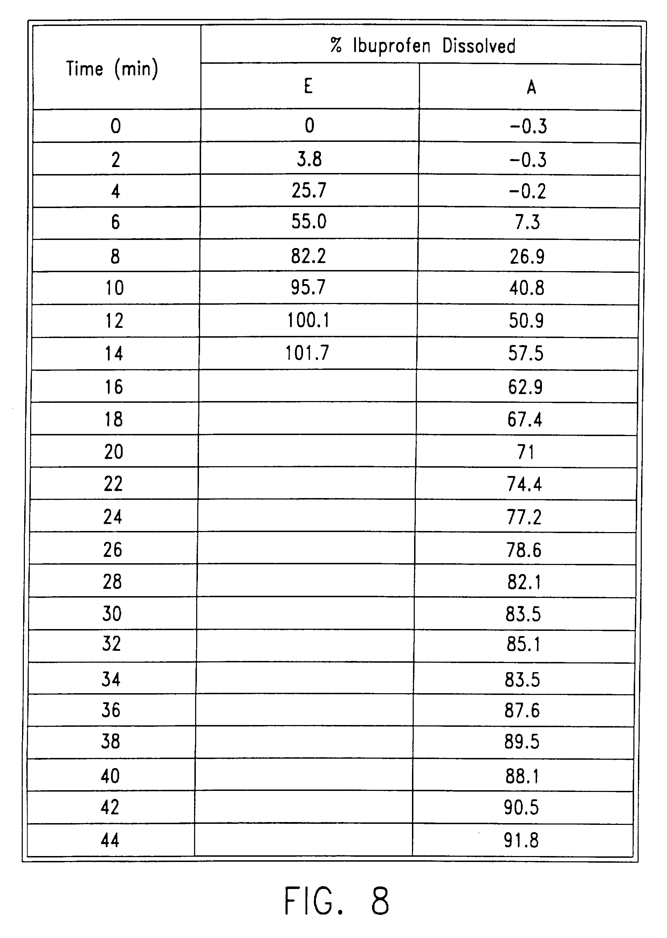

Figure 8 is a table showing comparative dissolution data for prior art 200 mg ibuprofen tablets (A - Advil®) and 200 mg ibuprofen tablets (E) including 1:1 ibuprofen-sodium starch glycolate mechanically activated composite. The dissolution method utilized USP Apparatus II, 50 rpm, pH 6.0 buffer 37 deg. C. The values shown in Figure 8 are the average values for six tablets. The tablets E were not film coated, but it is believed the absence of film coating may have caused only a negligible difference in the dissolution values. Figure 8 shows that tablets E have a substantially higher dissolution rate than tablets A.

Surprisingly, formation of the mechanically activated composite also provides taste retardation for pharmaceutically active substances that have strong taste. Sodium starch glycolate masks the taste of the active substance thereby minimizing or eliminating the need for a filler, flavor or sweetener, as required in prior art formulations.

To prepare the mechanically activated composite, an active substance and sodium starch glycolate, both in powder form, are mixed together in a solids mixer. The active substance and sodium starch glycolate both have a particle size distribution of between 0.01 and 1000 microns.

Although not necessary, the mixture may be heated under vacuum to a temperature compatible with the stability of the constituent substances to eliminate any foreign substances that may be present.

Heating can be done in the same chamber, in which the co-grinding is to be carried out, or outside the chamber.

The mixture is introduced into the grinding chamber together with the grinding media. A preferred grinding media is cylindrical in shape. Grinding is commenced. The high energy co-grinding is done for a time of between about 5 minutes and 48 hours, and preferably between about 5 minutes and 8 hours.

It has unexpectedly been found that sodium starch glycolate requires substantially less grinding time in order to provide substantially improved dissolution rates, solubilization kinetics, bioavailability, and peak drug levels in plasma of the active substance when compared with the active substance per se, which are comparable to or better than those obtained with other known support materials. With high energy grinding of sodium starch glycolate and active substances, a shorter co-grinding time is sufficient to attain the desired enhanced characteristics of the drug, with consequent considerable advantages in processing costs.

It also has unexpectedly been found that the desired results can be obtained without addition of a solvent or solvent vapor to the co-grinding process. In this respect also sodium starch glycolate as a support material differs advantageously from other known support materials, which generally require that a solvent be added to the grinding process. Although a solvent could be used, it is not necessary to add a solvent to the co-grinding process. Sodium starch glycolate is a hydrophilic polymer and typically contains some water which may be released into the atmosphere during the grinding process. It is also possible to achieve the results of the invention with dried or lower water content sodium starch glycolate.

After completion of the co-grinding operation, the mechanically activated composite that is obtained may be placed in an oven, or another similar apparatus, and may be dried, if necessary, at a temperature compatible with the stability of the constituent substances.

The mechanically activated composite may be sieved if necessary to eliminate any formed aggregates.

A preferred high energy grinding apparatus for a process of the invention is of the type based on high impact energy between the grinding media and the materials that are being ground. Such mills produce energy for grinding by the frequency of the vibration. In particular, high energy vibration mills using cylindrical grinding media are preferred. Although dependent upon the specific conditions involved, a high energy vibration mill provides about 10-1000 times the energy input of a conventional mill. Typically, a high energy grinding apparatus is utilized for batch processing and has a ratio of grinding load to horsepower of input energy from about 50 lbs./horsepower of input energy to about 400 lbs./horsepower of input energy. In contrast, a conventional milling apparatus typically processes between 800-1200 lbs./horsepower of input energy. Generally, the rpm of the motor for a high energy grinding apparatus is between about 400 rpm to about 1800 rpm, preferably between about 900 rpm to about 1500 rpm, and more preferably about 1200 rpm. The vertical amplitude of vibration of a high energy grinding apparatus is typically between about 1/8 inch to about 7/8 inch, and the lead angle is between about 0 degrees to about 90 degrees or between about 270 degrees to about 360 degrees.

Preferably, the high energy grinding apparatus includes controls for humidity and temperature of the materials that are being ground. The humidity and temperature of the materials are controlled to provide optimum conditions for forming the mechanically activated composite. Such optimum conditions include conditions at which the component materials of the composite are not adversely affected.

Sodium starch glycolate is sold commercially under the brand names Explotab®, Tablo® and Primogel®.

Although not necessary, a solvent that can be absorbed by sodium starch glycolate may be present in the sodium starch glycolate that is mixed with the active substance. By way of example, solvents suitable for use in the present invention are water, methylene chloride, methanol, ethanol, isopropanol and their mixtures.

Many drugs are suitable for formulation with a mechanically activated composite of the present invention, such as anti-inflammatories, analgesics, tranquilizers, sedatives, oral antitumor agents, steroids, etc.

Active substances that are particularly suitable for use in the present invention include ibuprofen, nifedipine and mefenamic acid. Other active substances include griseofulvin, piroxicam, diacerein, diltiazem, megestrol acetate, nicergoline and the like, ketoprofen, naproxen, diclofenac, lorazepam, oxazepam, etodolac, etc.

The weight ratio of sodium starch glycolate to the active substance is between about 100:1 and 0.3:1 and preferably between about 10:1 and 0.5:1.

Drugs according to the present invention can be used in the preparation of various pharmaceutical dosage forms such as tablets (of immediate or controlled release), chewable tablets, capsules (of immediate or controlled release), suspensions, transdermal films, and topical formulations, etc.

For formulating immediate-release tablets or capsules, the drugs can be mixed with excipients normally used in the pharmaceutical field, such as lactose, starch, calcium phosphate, microcrystalline cellulose etc. To formulate controlled-release tablets or capsules, the drugs can be mixed with polymers, such as methylcellulose and derivatives, polymethylmethacrylates, ethylcellulose etc.

The invention also envisions the formulation of combination drugs, such as codeine in combination with ibuprofen.

The characteristics of the formulations that are obtainable by a process of the invention can be determined by one or more of the following methods:
- dissolution rate;
- solubilization kinetics;
- differential scanning calorimetry to measure the heat of fusion, which is related to the residual crystallinity of the drug;
- differential scanning calorimetry or other thermoanalytical methods to evaluate the reduction in melting point.

The following examples are given for purposes of non-limiting illustration.

### EXAMPLE 1.

| **Ibuprofen 200 mg Tablet** | | | |
|---|---|---|---|
| Ingredient | | Preferred Quantity mg per tablet | Ranges (mg) |
| 1. | Ibuprofen USP | 200 | -- |
| 2. | Sodium Starch Glycolate, NF | 200 | 100 - 300 |
| 3. | Microcrystalline Cellulose | 30 | 10 - 90 |
| 4. | Crosscarmellose Sodium, NF | 50 | 5 - 100 |
| 5. | Colloidal Silicone Dioxide, NF | 5 | 1 - 50 |
| 6. | Stearic Acid, NF | 15 | 3 - 100 |
| 7. | Talc, NF | 0 | 0 - 50 |
| | Total Wt. | 500 | 450 - 650 |

Similarly, a 20 mg. dosage tablet also may be formulated.

### EXAMPLE 2.

| **Nifedipine 10 mg Capsule** | | |
|---|---|---|
| Ingredient | | Preferred Quantity (mg/cap) |
| 1. | Nifedipine-Sodium Starch Glycolate 1:3 Composite: | |
| | Nifedipine | 10.0 |
| | Sodium Starch Glycolate | 30.0 |
| 2. | Microcrystalline Cellulose | 0 - 70.0 |
| 3. | Lactose | 0 - 50.0 |
| 4. | Crosscarmellose Sodium | 0 - 20.0 |
| 5. | Magnesium Stearate | 0 - 1.0 |
| 6. | Gelatin Capsule | |
| | Total Fill Weight: | 100 - 150.0 |

The above formulation can also be compressed in a tablet form.

### EXAMPLE 3.

| **Mefenamic Acid 250 mg Capsule** | | |
|---|---|---|
| Ingredient | | Preferred Quantity (mg/cap) |
| 1. | Mefenamic Acid-Sodium Starch Glycolate 1:1 Composite: | |
| | Mefenamic Acid | 250.0 |
| | Sodium Starch Glycolate | 250.0 |
| 2. | Lactose | 0 - 50.0 |
| 3. | Crosscarmellose Sodium | 0 - 30.0 |
| 4. | Magnesium Stearate | 0 - 20.0 |
| 5. | Gelatin Capsule | |
| | Total Fill Weight: | 500 - 575.0 |

The above formulation can also be compressed in a tablet form.

### EXAMPLE 4.

| **Ibuprofen 200 mg and Codeine Phosphate 15 to 60 mg Tablets** | | |
|---|---|---|
| Ingredient | | Preferred Quantity (mg/tablet) |
| 1. | Ibuprofen-Sodium Starch Glycolate 1:1 Composite: | |
| | Ibuprofen | 200.0 |
| | Sodium Starch Glycolate | 200.0 |
| 2. | Codeine Phosphate | 15 to 60.0 |
| 3. | Microcrystalline Cellulose | 0 - 80.0 |
| 4. | Lactose | 0 - 30.0 |
| 5. | Crosscarmellose Sodium | 5 - 40.0 |
| 6. | Magnesium Stearate | 0 - 4.0 |
| | Total Fill Weight: | 450 - 575.0 |

### EXAMPLE 5.

| **Nifedipine 90 mg Sustained Release Tablets** | | |
|---|---|---|
| Ingredient | | Preferred Quantity (mg/tablet) |
| 1. | Nifedipine - Sodium Starch Glycolate 1:3 Composite: | |
| | Nifedipine | 90.0 |
| | Sodium Starch Glycolate | 90 - 270.0 |
| | Povidone | 0 - 15.0 |
| 2. | Ethylcellulose | 0 - 54.0 |
| 3. | Triethyl Citrate | 0 - 27.0 |
| 4. | Dibutyl Sebacate | 0 - 27.0 |
| 5. | Ethyl acrylate and Methyl methacrylate copolymer | 0 - 54.0 |
| 6. | Hydroxypropylmethyl cellulose | 0 - 10.0 |
| 7. | Hydroxypropyl cellulose | 0 - 10.0 |
| 8. | Polyethylene glycol | 0 - 24.0 |
| 9. | Talc | 0 - 30.0 |
| 10. | Microcrystalline Cellulose | 0 - 360.0 |
| 11. | Lactose | 0 - 50.0 |
| 12. | Crosscarmellose Sodium | 0 - 80.0 |
| 13. | Stearic Acid | 0 - 40.0 |
| 14. | Magnesium Stearate | 0 - 12.0 |
| | Total Fill Weight: | 600 - 800.0 |

The above formulation can be linearly scaled down to compress tablets containing 30 or 60 mg of nifedipine.

Although the present invention has been described in relation to particular embodiments thereof, many other variations and modifications and other uses will become apparent to those skilled in the art.

## Claims

1. A mechanically activated high energy co-ground composite of a pharmaceutically active substance and sodium starch glycolate characterized by an increased dissolution rate and increased solubilization kinetics of the active substance compared to the active substance per se.

2. The mechanically activated composite as claimed in claim 1, wherein the composite is characterized by increased bioavailability of the pharmaceutically active substance compared to the active substance per se.

3. The mechanically activated composite as claimed in claim 1, wherein the composite is characterized by increased peak drug levels in plasma of the pharmaceutically active substance compared to the active substance per se.

4. The mechanically activated composite as claimed in claim 1, wherein the composite is characterized by a faster peak level of therapeutic effect of the pharmaceutically active substance compared to the active substance per se.

5. The mechanically activated composite as claimed in claim 1, wherein the pharmaceutically active substance is selected from the group consisting of antiinflammatories, analgesics, tranquilizers, sedatives, oral antitumor agents, and steroids.

6. The mechanically activated composite as claimed in claim 5, wherein the pharmaceutically active substance is poorly soluble.

7. The mechanically activated composite as claimed in claim 5, wherein the pharmaceutically active substance is ibuprofen.

8. The mechanically activated composite as claimed in claim 4, wherein the pharmaceutically active substance is nifedipine.

9. The mechanically activated composite as claimed in claim 4, wherein the pharmaceutically active substance is mefenamic acid.

10. The mechanically activated composite as claimed in claim 1, wherein the pharmaceutically active substance is poorly soluble.

11. The mechanically activated composite as claimed in claim 1, wherein the characteristic taste of the pharmaceutically active substance is masked.

12. The mechanically activated composite as claimed in claim 1, wherein the weight ratio of the sodium starch glycolate to the pharmaceutically active substance is between about 100:1 and 0.3:1.

13. The mechanically activated composite as claimed in claim 12, wherein the weight ratio of the sodium starch glycolate to the pharmaceutically active substance is between about 10:1 and 0.5:1.

14. The mechanically activated composite as claimed in claim 1, wherein the pharmaceutically active substance and sodium starch glycolate are co-ground in the absence of any other solvent.

15. A pharmaceutical composition comprising the mechanically activated composite of claim 1 and an excipient therefor.

16. The pharmaceutical composition as claimed in claim 15, wherein the composition is chewable.

17. The pharmaceutical composition as claimed in claim 15, wherein the characteristic taste of the pharmaceutically active substance is masked.

18. The pharmaceutical composition as claimed in claim 15, further comprising codeine.

19. A process for manufacturing a mechanically activated composite, comprising the step of:
high energy co-grinding a mixture of a pharmaceutically active substance and sodium starch glycolate for a time sufficient to obtain a mechanically activated composite of the pharmaceutically active substance and the sodium starch glycolate characterized by an increased dissolution rate and increased solubilization kinetics of the active substance compared to the active substance per se.

20. The process as claimed in claim 19, wherein the co-grinding is done for a time period between about 5 minutes and 48 hours.

21. The process as claimed in claim 20, wherein the co-grinding is done for a time period not more than about 8 hours.

22. The process as claimed in claim 19, further comprising the step of controlling temperature and humidity of the pharmaceutically active substance and sodium starch glycolate to provide optimum process conditions.

23. The process as claimed in claim 19, wherein the pharmaceutically active substance and sodium starch glycolate are co-ground in the absence of any other solvent.

24. The process as claimed in claim 19, wherein the high energy co-grinding is done by a vibration mill.

25. The process as claimed in claim 24, wherein the vibration mill further comprises means for controlling temperature and humidity.

26. The process as claimed in claim 24, wherein the vibration mill is adapted for forming the mechanically activated composite in a time period between about 5 minutes and 48 hours.

27. The process as claimed in claim 26, wherein the vibration mill is adapted for forming the mechanically activated composite in a time period not more than about 8 hours.

28. A method of masking the taste of a pharmaceutically active substance, comprising the step of:
high energy co-grinding a mixture of a pharmaceutically active substance and sodium starch glycolate for a time sufficient to obtain a mechanically activated composite of the pharmaceutically active substance and the sodium starch glycolate characterized by an increased dissolution rate and increased solubilization kinetics compared to the active substance per se, whereby the characteristic taste of the active substance is masked.

## Patentansprüche

1. Mechanisch aktiviertes, mit hoher Energie vermahlenes Composite aus einer pharmazeutisch aktiven Substanz und Natriumstärkeglycolat, das durch, verglichen mit der aktiven Substanz per se, erhöhte Auflösungsgeschwindigkeit und erhöhte Solubilisierungskinetik der aktiven Substanz **gekennzeichnet** ist.

2. Mechanisch aktiviertes Composite nach Anspruch 1, wobei das Composite durch, verglichen mit der aktiven Substanz per se, erhöhte Bioverfügbarkeit der pharmazeutisch aktiven Substanz **gekennzeichnet** ist.

3. Mechanisch aktiviertes Composite nach Anspruch 1, wobei das Composite durch, verglichen mit der aktiven Substanz per se, erhöhte Spitzenarzneimittelkonzentrationen der pharmazeutisch aktiven Substanz im Blutplasma **gekennzeichnet** ist.

4. Mechanisch aktiviertes Composite nach Anspruch 1, wobei das Composite durch, verglichen mit der aktiven Substanz per se, ein schnelles Spitzenniveau des therapeutischen Effektes der pharmazeutisch aktiven Substanz **gekennzeichnet** ist.

5. Mechanisch aktiviertes Composite nach Anspruch 1, wobei die pharmazeutisch aktive Substanz aus der Gruppe bestehend aus entzündungshemmenden Mitteln, Schmerzmitteln, Tranquilizern, Sedativa, oralen Antitumormitteln und Steroiden ausgewählt ist.

6. Mechanisch aktiviertes Composite nach Anspruch 5, wobei die pharmazeutisch aktive Substanz schlecht löslich ist.

7. Mechanisch aktiviertes Composite nach Anspruch 5, wobei die pharmazeutisch aktive Substanz Ibuprofen ist.

8. Mechanisch aktiviertes Composite nach Anspruch 4, wobei die pharmazeutisch aktive Substanz Nifedipin ist.

9. Mechanisch aktiviertes Composite nach Anspruch 4, wobei die pharmazeutisch aktive Substanz Mefenaminsäure ist.

10. Mechanisch aktiviertes Composite nach Anspruch 1, wobei die pharmazeutisch aktive Substanz schlecht löslich ist.

11. Mechanisch aktiviertes Composite nach Anspruch 1, wobei der charakteristische Geschmack der pharmazeutisch aktiven Substanz maskiert ist.

12. Mechanisch aktiviertes Composite nach Anspruch 1, wobei das Gewichtsverhältnis des Natriumstärkeglycolats zu der pharmazeutisch aktiven Substanz zwischen etwa 100:1 und 0,3:1 liegt.

13. Mechanisch aktiviertes Composite nach Anspruch 12, wobei das Gewichtsverhältnis des Natriumstärkeglycolats zu der pharmazeutisch aktiven Substanz zwischen etwa 10:1 und 0,5:1 liegt.

14. Mechanisch aktiviertes Composite nach Anspruch 1, wobei die pharmazeutisch aktive Substanz und Natriumstärkeglycolat in Abwesenheit eines anderen Lösungsmittels zusammen vermahlen sind.

15. Pharmazeutische Zusammensetzung, die das mechanisch aktivierte Composite nach Anspruch 1 und eine Trägersubstanz dafür umfaßt.

16. Pharmazeutische Zusammensetzung nach Anspruch 15, wobei die Zusammensetzung kaubar ist.

17. Pharmazeutische Zusammensetzung nach Anspruch 15, wobei der charakteristische Geschmack der pharmazeutisch aktiven Substanz maskiert ist.

18. Pharmazeutische Zusammensetzung nach Anspruch 15, die außerdem Codein enthält.

19. Verfahren zur Herstellung eines mechanisch aktivierten Composites, umfassend den Schritt:
Vermahlen eines Gemisches aus einer pharmazeutisch aktiven Substanz und Natriumstärkeglycolat mit hoher Energie über eine Zeit, die ausreicht, um ein mechanisch aktiviertes Composite aus der pharmazeutisch aktiven Substanz und dem Natriumstärkeglycolat zu erhalten, das durch, verglichen mit der aktiven Substanz per se, erhöhte Auflösungsgeschwindigkeit und erhöhte Solubilisierungskinetik der aktiven Substanz **gekennzeichnet** ist.

20. Verfahren nach Anspruch 19, wobei das Vermahlen über einen Zeitraum zwischen etwa 5 min und 48 h durchgeführt wird.

21. Verfahren nach Anspruch 20, wobei das Vermahlen über einen Zeitraum von nicht mehr als etwa 8 h durchgeführt wird.

22. Verfahren nach Anspruch 19, das außerdem den Schritt des Steuerns von Temperatur und Feuchtigkeit der pharmazeutisch aktiven Substanz und des Natriumstärkeglycolats unter Bereitstellung optimaler Verfahrensbedingungen umfaßt.

23. Verfahren nach Anspruch 19, wobei die pharmazeutisch aktive Substanz und das Natriumstärkeglycolat in Abwesenheit eines anderen Lösungsmittels zusammen vermahlen werden.

24. Verfahren nach Anspruch 19, wobei das Vermahlen mit hoher Energie mit einer Schwingmühle durchgeführt wird.

25. Verfahren nach Anspruch 24, wobei die Schwingmühle außerdem Mittel zur Steuerung von Temperatur und Feuchtigkeit umfaßt.

26. Verfahren nach Anspruch 24, wobei die Schwingmühle zur Bildung des mechanisch aktivierten Composites in einem Zeitraum zwischen etwa 5 min und 48 h angepaßt ist.

27. Verfahren nach Anspruch 26, wobei die Schwingmühle zur Bildung des mechanisch aktivierten Composites in einem Zeitraum von nicht mehr als etwa 8 h angepaßt ist.

28. Verfahren zur Maskierung des Geschmacks einer pharmazeutisch aktiven Substanz, umfassend den Schritt:
Vermahlen eines Gemisches aus einer pharmazeutischen aktiven Substanz und Natriumstärkeglycolat mit hoher Energie über einen Zeitraum, der ausreicht, ein mechanisch aktiviertes Composite aus der pharmazeutisch aktiven Substanz und dem Natriumstärkeglycolat zu erhalten, das durch, verglichen mit der aktiven Substanz per se, erhöhte Auflösungsgeschwindigkeit und erhöhte Solubilisierungskinetik der aktiven Substanz **gekennzeichnet** ist, wodurch der charakteristische Geschmack der aktiven Substanz maskiert wird.

## Revendications

1. Composé à forte énergie activé mécaniquement composite d'une substance pharmaceutiquement active et de glycolate d'amidon sodique, caractérisé par une augmentation de la vitesse de dissolution et de la cinétique de solubilisation de la substance active par rapport à la substance active en soi.

2. Composite activé mécaniquement selon la revendication 1, qui se caractérise par une augmentation de la biodisponibilité de la substance pharmaceutiquement active par rapport à la substance active en soi.

3. Composite activé mécaniquement selon la revendication 1, qui se caractérise par une augmentation des concentrations maximales en médicament dans le plasma de la substance pharmaceutiquement active par rapport à la substance active en soi.

4. Composite activé mécaniquement selon la revendication 1, qui se caractérise par un niveau maximal plus rapide de l'effet thérapeutique de la substance pharmaceutiquement active par rapport à la substance active en soi.

5. Composite activé mécaniquement selon la revendication 1, dans lequel la substance pharmaceutiquement active est choisie dans le groupe constitué par les anti-inflammatoires, les analgésiques, les tranquillisants, les sédatifs, les agents antitumoraux oraux et les stéroïdes.

6. Composite activé mécaniquement selon la revendication 5, dans lequel la substance pharmaceutiquement active est faiblement soluble.

7. Composite activé mécaniquement selon la revendication 5, dans lequel la substance pharmaceutiquement active est l'ibuprofène.

8. Composite activé mécaniquement selon la revendication 4, dans lequel la substance pharmaceutiquement active est la nifédipine.

9. Composite activé mécaniquement selon la revendication 4, dans lequel la substance pharmaceutiquement active est l'acide méfénamique.

10. Composite activé mécaniquement selon la revendication 1, dans lequel la substance pharmaceutiquement active est faiblement soluble.

11. Composite activé mécaniquement selon la revendication 1, dans lequel le goût caractéristique de la substance pharmaceutiquement active est masqué.

12. Composite activé mécaniquement selon la revendication 1, dans lequel le rapport en poids du glycolate d'amidon sodique sur la substance pharmaceutiquement active est de 100/1 environ à 0,3/1.

13. Composite activé mécaniquement selon la revendication 12, dans lequel le rapport en poids du glycolate d'amidon sodique sur la substance pharmaceutiquement active est de 10/1 environ à 0,5/1.

14. Composite activé mécaniquement selon la revendication 1, dans lequel la substance pharmaceutiquement active et le glycolate d'amidon sodique sont co-broyés en l'absence de tout autre solvant.

15. Composition pharmaceutique comprenant le composite activé mécaniquement de la revendication 1 et un excipient pour celui-ci.

16. Composition pharmaceutique selon la revendication 15, qui est à croquer.

17. Composition pharmaceutique selon la revendication 15, dans laquelle le goût caractéristique de la substance pharmaceutiquement active est masqué.

18. Composition pharmaceutique selon la revendication 15, comprenant en outre de la codéine.

19. Procédé de fabrication d'un composite activé mécaniquement, comprenant l'étape de :
co-broyage à haute énergie d'un mélange d'une substance pharmaceutiquement active et de glycolate d'amidon sodique suffisamment longtemps pour obtenir un composite activé mécaniquement de la substance pharmaceutiquement active et du glycolate d'amidon sodique caractérisé par une augmentation de la vitesse de dissolution et de la cinétique de solubilisation de la substance active par rapport à la substance active en soi.

20. Procédé selon la revendication 19, dans lequel le co-broyage est effectué pendant une durée de 5 minutes environ à 48 heures.

21. Procédé selon la revendication 20, dans lequel le co-broyage ne dure pas plus de 8 heures environ.

22. Procédé selon la revendication 19, comprenant en outre l'étape de régulation de la température et de l'humidité de la substance pharmaceutiquement active et du glycolate d'amidon sodique afin d'offrir des conditions de procédé optimales.

23. Procédé selon la revendication 19, dans lequel la substance pharmaceutiquement active et le glycolate d'amidon sodique sont co-broyés en l'absence de tout autre solvant.

24. Procédé selon la revendication 19, dans lequel le co-broyage à haute énergie est effectué par un broyeur à vibrations.

25. Procédé selon la revendication 24, dans lequel le broyeur à vibrations comprend en outre des moyens de régulation de la température et de l'humidité.

26. Procédé selon la revendication 24, dans lequel le broyeur à vibrations permet de former le composite activé mécaniquement dans un délai de 5 minutes à 48 heures.

27. Procédé selon la revendication 26, dans lequel le broyeur à vibrations permet de former le composite activé mécaniquement dans un délai ne dépassant pas 8 heures environ.

28. Procédé de masquage du goût d'une substance pharmaceutiquement active, comprenant l'étape de :
co-broyage à haute énergie d'un mélange d'une substance pharmaceutiquement active et de glycolate d'amidon sodique suffisamment longtemps pour obtenir un composite activé mécaniquement de la substance pharmaceutiquement active et du glycolate d'amidon sodique caractérisé par une augmentation de la vitesse de dissolution et de la cinétique de solubilisation par rapport à la substance active en soi, au moyen duquel le goût caractéristique de la substance active est masqué.
